# EUROPEAN PATENT APPLICATION

(11) **EP 4 744 700 A1**
(43) Date of publication of application: **20.05.2026**
(21) Application number: 25215452.1
(22) Date of filing: 13.11.2025
(51) Int. Cl.: A61M 5/34

(54) **LAYER BONDING GLASS SYRINGE**

(30) Priority: 19.11.2024 US 202463722227 P
(71) Applicant: ATS Corporation, Cambridge, Ontario N3H 4R7 (CA)
(72) Inventor: LINDNER, Roland, 85551 Heimstetten (DE)
(74) Representative: Bardehle Pagenberg Partnerschaft mbB Patentanwälte Rechtsanwälte

(57) **Abstract**

This invention relates to a medical glass syringe, comprising: a glass barrel and a needle, the glass barrel comprising: a needle hub, wherein the needle is fixed by means of a first adhesive, and wherein the exit end of the needle hub is provided with a second adhesive and a method of treating a medical glass syringe.

## Description

### 1. Field of the invention

This invention relates to a medical glass syringe, and a method of treating a medical glass syringe.

### 2. Background

The invention relates to medical glass syringes, specifically focusing on the secure attachment of the needle to the glass barrel. In medical applications, syringes must be reliable, ensuring that the needle is firmly fixed to prevent detachment, leakage, or contamination of the drug inside. The process of attaching the needle typically involves adhesives, which must be carefully chosen and applied to meet the stringent requirements of medical use, including compatibility with drugs, resistance to environmental factors, and proper curing under manufacturing conditions.

The primary problem addressed by this invention is ensuring the secure and reliable attachment of the needle to the glass barrel while maintaining the integrity of the drug inside the syringe. Traditional methods might suffer from issues like improper adhesive curing, insufficient bond strength, or contamination risks due to interaction between the drug and the adhesive. Additionally, curing adhesives in a controlled manner can be challenging, especially when different parts of the syringe are exposed to varying levels of radiation during the curing process. Thus, there is a demand for improved medical glass syringes.

It is thus an object of the present invention to overcome some or all the deficiencies of the prior art. This invention provides a solution by utilizing a combination of adhesives with specific properties-such as differing viscosities, optical characteristics, and curing behaviors-tailored to optimize the syringe's performance. The invention also addresses the problem of ensuring that only certain adhesives come into contact with the drug, thereby maintaining the drug's purity and efficacy.

### 3. Summary

The above objects are at least partially achieved by the subject matter of independent claim 1. Preferred embodiments are the subject of the dependent claims, and the skilled person will find clues to other suitable aspects of the present invention in the overall disclosure of the present application.

An aspect of the invention relates to a medical glass syringe, comprising a glass barrel and a needle, the glass barrel comprising a needle hub, wherein the needle is fixed by means of a first adhesive, and wherein the exit end of the needle hub is provided with a second adhesive. It is customary in the art that a needle is a slender, hollow, metal tube attached to a syringe, used for injecting fluids into or withdrawing fluids from a body. It typically has a sharp, pointed end for piercing skin and a hollow interior through which the fluid flows. A glass barrel is the main cylindrical body of the syringe made of glass, which holds and contains the medical drug or fluid to be injected. A glass barrel is generally transparent, allowing visual inspection of the contents and dosage, and is designed to be chemically inert to prevent interaction with the drug. The term interior space relates to the internal cavity within the glass barrel where the medical drug or fluid is stored. This space is isolated from the external environment and is in communication with the needle, allowing for the controlled dispensing of the contents through the needle. A needle hub is the component that connects the needle to the glass barrel of the syringe. It provides a secure interface for attaching the needle and can incorporate adhesives to bond the needle to the syringe. The exit end of the needle hub is the terminal part of the needle hub from which the needle protrudes. It is the point where the needle exits the hub and extends outward, e.g. for injection. The exit end may be sealed and reinforced with adhesives to provide a secure and leak-proof connection between the needle and the syringe.

In such a syringe, the use of two different adhesives ensures that the needle is securely attached to the syringe, minimizing the risk of detachment during use. The first adhesive firmly fixes the needle to the hub, while the second adhesive at the exit end provides an additional layer of security, potentially preventing leakage of medical fluids. This dual adhesive system can enhance the safety and reliability of the syringe. This way, the first adhesive can be chosen by providing an optimized compatibility with drugs inside the syringe, while the second adhesive can be chosen by providing optimized fixation of the needle without needing to take the drug compatibility of the second adhesive into account, as it is not in contact with the drug inside the syringe.

Further improvement can be achieved, when the second adhesive has generally no oxygen inhibiting properties and/or wherein the first adhesive has oxygen inhibiting properties.

In such an embodiment, oxygen inhibition refers to the interaction between the adhesive and oxygen at the surface. When the adhesive with oxygen-inhibiting properties is exposed to air, oxygen can prevent the formation of long-chain polymerization: a process where molecules link together to form a strong, durable structure. Instead, only short-chain oligomers are formed. When the first adhesive comes into contact with oxygen, the oxygen binds to the free radicals that are necessary for long-chain polymerization, which is the process where molecules link together to form a strong and durable bond. Because of this, only short-chain oligomers are formed, which are shorter and less stable molecular chains. These oligomers cannot undergo any further light-stimulated reactions, leading to the formation of a gel-like, wet layer on the surface of the adhesive.

Further improvement is achieved, when the second adhesive has a lower viscosity than the first adhesive in uncured condition.

In such an embodiment, the lower viscosity of the second adhesive allows it to flow more easily into small gaps and spaces at the exit end of the needle hub, ensuring a thorough and even application. This characteristic helps create a secure seal while avoiding excess adhesive buildup, which could interfere with the syringe's function. The higher viscosity of the first adhesive provides a stronger bond where it is most needed to hold the needle firmly in place. This combination of adhesives with different viscosities enhances the overall assembly process and ensures the syringe's reliability during medical procedures.

Further improvement is achieved when the first and the second adhesive are curable with radiation of the same wavelength, wherein the wavelength of the radiation is in the range 320 nm to 410 nm, preferably in the range 340 nm to 390 nm, most preferably around 365 nm.

In such an embodiment curing both adhesives with the same wavelength of radiation simplifies the manufacturing process, as only one type of curing equipment is needed. The specific wavelength range ensures effective curing without damaging the syringe materials. The preferred wavelength of for instance 365 nm is commonly used in UV curing processes. This uniform curing method enhances production speed and consistency, ensuring that the adhesives bond properly, resulting in a high-quality, durable syringe suitable for important medical applications.

Further improvement is achieved when the first and the second adhesive have the same optical properties when cured.

In such an embodiment, using adhesives with the same optical properties ensures a uniform appearance of the syringe after curing. This uniformity is not only aesthetically demanded but also beneficial for quality control, as any inconsistencies in the bonding process would be more noticeable. Additionally, matching optical properties can prevent issues like light refraction or reflection at the adhesive joints, which could interfere with the syringe's function or inspection processes. This design choice enhances both the visual quality and performance reliability of the syringe.

Further improvement is achieved when, the weight ratio between the first and the second adhesive is in the range of 0.2 to 1.8, preferably in the range of 0.6 to 1.4, most preferably 1, when uncured.

In such an embodiment, setting the weight ratio of the adhesives ensures that the right amount of each adhesive is used to achieve optimal bonding and performance. The most preferred ratio of 1 indicates that equal amounts of both adhesives are used, which can simplify the manufacturing process and ensure consistency. By controlling this ratio, the syringe can maintain a balance between the strength provided by the first adhesive and the flexibility or flow properties of the second adhesive. This careful balance enhances the syringe's durability and functionality, making it suitable for medical applications that require precise and reliable performance.

Further improvement is achieved when the second adhesive forms a dome-shaped structure on the exit end and wherein the height of the dome-shaped structure is preferably in the range of 1 mm to 5 mm, more preferably in the range of 2 mm to 4 mm, and most preferably around 3 mm and wherein the diameter of the dome-shaped structure is preferably in the range of 2 mm to 8 mm, more preferably in the range of 4 mm to 6 mm, and most preferably about 5 mm.

In such an embodiment, the dome-shaped structure created by the second adhesive provides a smooth, protective cover over the exit end of the needle hub. The specified dimensions ensure that the dome is large enough to offer protection and secure sealing, yet small enough to avoid interfering with the syringe's use. This design helps to prevent contamination, reduce the risk of injury from the needle, and enhance the overall safety and usability of the syringe. The carefully controlled dimensions of the dome can ensure consistent manufacturing quality and reliability.

Further improvement is achieved when the shaft of the needle passes through the dome-shaped structure.

In such an embodiment, having the needle shaft pass through the dome-shaped adhesive structure ensures that the needle is securely encased and stabilized at the exit point. This design can provide additional support and protection for the needle, reducing the likelihood of bending or breakage during use. The dome-shaped structure also acts as a seal, preventing any leakage of fluids around the needle and enhancing the overall safety and hygiene of the syringe.

Further improvement can be achieved when the first adhesive is LOCTITE^{®} AA 3345 and the second adhesive is preferably LOCTITE^{®} AA 3942.

In such an embodiment, using LOCTITE^{®} AA 3345 as the first adhesive provides strong bonding properties, ideal for securely fixing the needle to the hub; it is furthermore a well-known adhesive, which is approved for using in contact with medicines. LOCTITE^{®} AA 3942, used as the second adhesive, is selected for its specific characteristics that suit the application, such as its flow properties and curing behavior. These adhesives are well-known for their reliability in medical applications, ensuring that the syringe components are bonded with high strength and durability.

Further improvement is achieved when the first adhesive is in communication with the interior space of the glass barrel and wherein the second adhesive is not in communication with the interior space of the glass barrel.

In such an embodiment, having the first adhesive in communication with the interior space of the glass barrel ensures that it securely seals and bonds the needle to the syringe in a way that prevents fluid leakage from the inside. This is important for maintaining the sterility and integrity of the medication within the syringe. The second adhesive, not being in contact with the interior, is applied externally, likely serving as an additional seal or protective layer without risking contamination of the syringe's contents. This design optimizes the syringe's safety and performance, making it especially suitable for medical procedures that require precise fluid handling and strict hygiene standards.

Further improvement is achieved when the first adhesive is arranged close to the interior space of the glass barrel, such that a medical drug inside the medical syringe is in contact with the first adhesive and not with the second adhesive.

In such an embodiment, the first adhesive's placement near the interior ensures that it directly interacts with the medical drug, providing a secure seal that keeps the drug contained without leakage. By keeping the second adhesive out of contact with the drug, the design minimizes any potential interactions between the drug and the adhesive materials, which could affect the drug's stability or efficacy. This arrangement is beneficial in preserving the purity and effectiveness of the medication.

Further improvement is achieved when the barrel and the needle hub are integrally formed with each other.

In such an embodiment, integrating the barrel and the needle hub into a single unit eliminates the need for separate parts and additional bonding processes, reducing the risk of leakage or detachment. This design enhances the structural integrity of the syringe, making it more robust and reliable. The seamless connection between the barrel and the hub also improves the manufacturing process by simplifying assembly and reducing potential points of failure.

Further improvement is achieved when the first adhesive is arranged in the needle hub as to extend to the exit end of the needle hub, and as to be flush with the surface of the exit end; and wherein preferably the second adhesive completely covers the first adhesive at the surface of the exit end.

In such an embodiment, the first adhesive extends to the exit end of the needle hub, providing a strong bond right up to the point where the needle exits the syringe. By being flush with the surface, it ensures a smooth and even application area for the second adhesive. The second adhesive, which completely covers the first adhesive, adds an extra layer of protection and sealing at the exit end. This dual-layer approach enhances the overall strength and durability of the syringe while minimizing any risk of fluid leakage. The coverage by the second adhesive also improves the syringe's resistance to environmental factors, making it effective in maintaining sterility and integrity during medical procedures.

Another aspect of the invention is a method of treating a medical glass syringe in accordance with any one of the preceding embodiments, comprising the curing of the first and the second adhesive by means of radiation.

Such a method involves exposing the syringe to a specific type of radiation, such as UVA light, which triggers the adhesives to cure and securely bond the needle to the glass barrel. This curing process ensures that the adhesives achieve their full bonding strength, resulting in a durable and reliable syringe. Using radiation to cure both adhesives simultaneously can streamline the manufacturing process, improving efficiency and consistency. This method is advantageous in producing high-quality medical syringes that meet stringent safety and performance standards.

This method can be improved when the means of radiation does not comprise a mercury vapour lamp and wherein the means of radiation preferably comprises a LED, the LED preferably configured to emitting UVA radiation.

In one preferred embodiment, using an LED to emit UVA radiation provides a safer and more energy-efficient alternative to traditional mercury vapor lamps. LEDs are more environmentally friendly, as they do not contain hazardous materials like mercury. Additionally, LEDs offer more precise control over the wavelength and intensity of the radiation, which can improve the curing process by ensuring that the adhesives cure uniformly and effectively. This method enhances the production quality of the syringe, making it more reliable and safer for medical use, while also aligning with modern sustainability practices.

### 4. Brief description of the figures

In the following, preferred embodiments of the disclosure are disclosed by reference to the accompanying figures.
- Fig. 1:: illustrates a medical glass syringe according to the invention in a side view.
- Fig. 2:: shows a medical glass syringe when exposed to UVA radiation in a side view.
- Fig. 3:: shows a medical glass syringe when only the first adhesive is applied in a schematic view.
- Fig. 4:: illustrates the medical glass syringe of Figure 3 with the second adhesive in a schematic view.

### 5. Detailed description of the figures

The subsequent sections provide a detailed description of the invention, referencing the accompanying illustrations for clarity. The descriptions represent examples only and are not intended to limit the invention's scope. Identical reference numerals across the figures and text denote the same components. The illustrations may not reflect actual size or scale; their dimensions, proportions, and depictions of elements might be enhanced for better understanding and visual convenience.

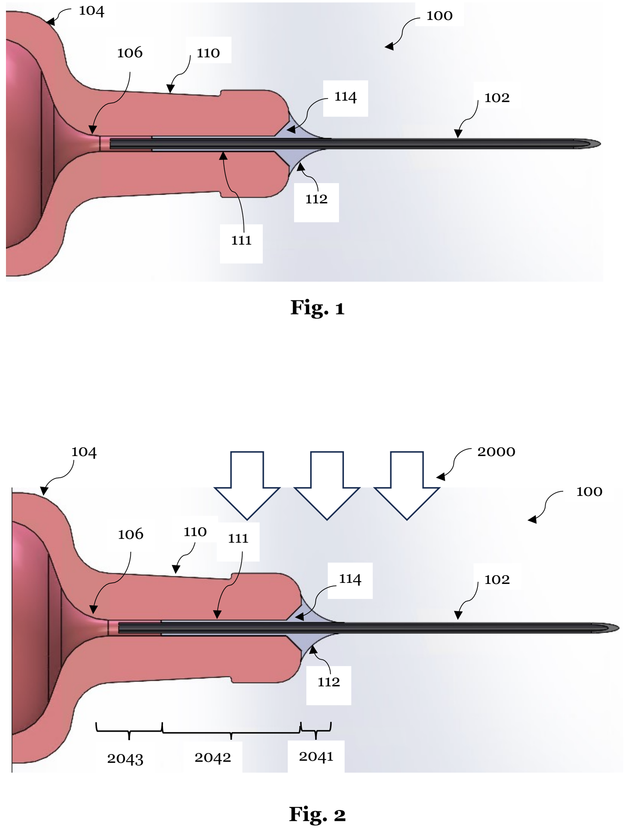

Figure 1 illustrates a medical glass syringe according to the invention in a side view. The medical glass syringe 100 comprises a glass barrel 104 with a needle hub 110. The needle 102 is fixed in the needle hub 110 by means of a first adhesive 111, such that the first adhesive 111 is in communication with the interior space 106 of the medical glass syringe. The second adhesive 112 forms a dome-shaped structure at the exit of the needle hub 114 and additionally fixes the needle 102. This way, both the first and the second adhesive 111,112 fix the needle but only the first adhesive 111 can be in contact with a drug at the interior space 106 inside the glass barrel 104.

The use of both adhesives can ensure a robust and reliable fixation of the needle to the syringe. The first adhesive 111, being in contact with the drug, is selected for its compatibility with medical substances and its ability to prevent leaks into the interior space 106. The second adhesive 112, forming a dome-shaped structure at the needle's exit, provides additional security by covering and protecting the junction where the needle exits the hub. This dual adhesive approach enhances the syringe's safety and reliability, maintaining drug purity and preventing needle detachment.

Figure 2 shows a medical glass syringe when exposed to UVA radiation in a side view. The medical glass syringe 100 comprises a glass barrel 104 with a needle hub 110. The needle 102 is fixed in the needle hub 110 by means of a first adhesive 111, such that the first adhesive 111 is in communication with the interior space 106 of the medical glass syringe. The second adhesive 112 forms a dome-shaped structure at the exit of the needle hub 114 and additionally fixes the needle 102. This way, both the first and the second adhesive 111,112 fix the needle but only the first adhesive 111 can be in contact with a drug at the interior space 106 inside the glass barrel 204. UVA radiation 2000 can penetrate the syringe completely, but the glass acts as a UV filter. UV curing should primarily focus on Zone C 2043, where the first adhesive 111 can be in direct contact with a drug inside the syringe. Since UVA radiation 2000 can pass through the second adhesive 112 in Zone A 2041 without a filtering effect of the glass, an imbalance in exposure occurs: When the first and the second adhesive 111,112 are the same, Zone C 2043 receives too little exposure, while Zone A 2041 tends to be overexposed.

By choosing differing first and second adhesives 111,112, this imbalance can be addressed. The first adhesive can be chosen for its ability to cure effectively under filtered UVA conditions in Zone C 2043, while the second adhesive can be selected to handle direct UVA exposure in Zone A 2041 without over-curing. This approach ensures that both adhesives cure properly, resulting in a syringe that is robust and reliable, without any over- and/or uncured adhesive residues.

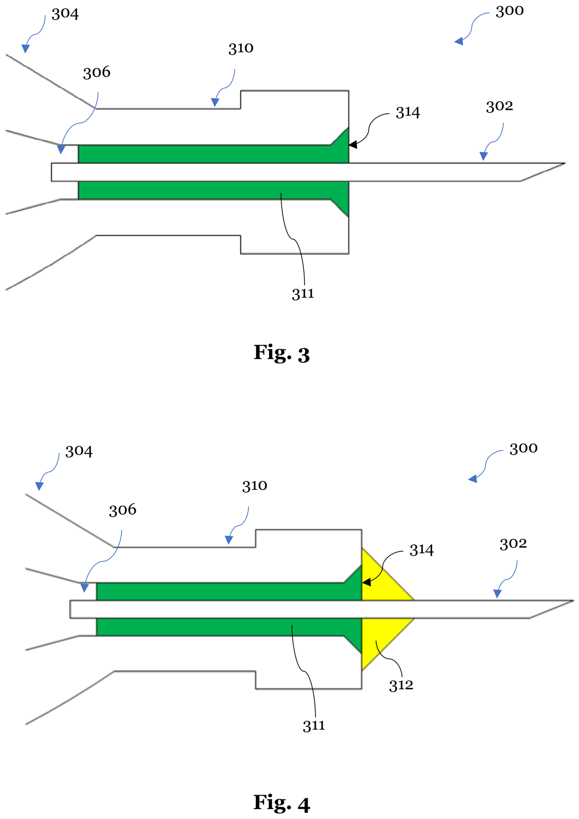

Figure 3 shows a medical glass syringe when only the first adhesive is applied in a schematic view. The medical glass syringe 300 comprises a glass barrel 304 with a needle hub 310. The needle 302 is fixed in the needle hub 310 by means of a first adhesive 311, such that the first adhesive 311 is in communication with the interior space 306 of the medical glass syringe. The first adhesive 311 is arranged flush with the surface of the exit of the needle hub 314.

By positioning the first adhesive 311 flush with the surface of the needle hub's exit 314, the design minimizes any gaps or irregularities. This approach simplifies the syringe construction, making it easier to manufacture while still ensuring that the needle is firmly attached.

Figure 4 illustrates the medical glass syringe of Figure 3 with the second adhesive in a schematic view. Figure 3 shows a medical glass syringe when only the first adhesive is applied in a schematic view. The medical glass syringe 300 comprises a glass barrel 304 with a needle hub 310. The needle 302 is fixed in the needle hub 310 by means of a first adhesive 311, such that the first adhesive 111 is in communication with the interior space 306 of the medical glass syringe. The first adhesive 311 is arranged flush with the surface of the exit of the needle hub 314. The second adhesive 312 is applied at the exit of the needle hub 314 and forms a dome-shaped structure, surrounding the needle 302 and completely covering the first adhesive 311 at the surface of the exit end 314.

The of the second adhesive 312 provides extra security and protection for the needle 302. The dome-like structure created by the second adhesive helps to further stabilize the needle at the exit point, reducing the risk of needle movement or detachment. This structure also acts as a protective barrier, potentially preventing contaminants from entering the syringe and ensuring that the needle remains securely in place during use.

### Reference list:

100, 300: medical glass syringe
102, 302: needle
104, 304: glass barrel
106, 306: interior space
110,310: needle hub
114, 314: exit of needle hub
111, 311: first adhesive
112, 312: second adhesive
2000: UVA radiation
2041: Zone A
2042: Zone B
2043: Zone C

## Claims

1. A medical glass syringe (100, 300), comprising:
a glass barrel (104, 304) and a needle (102, 302), the glass barrel (104, 304) comprising:
a needle hub (110, 310), wherein the needle (102, 302) is fixed by means of a first adhesive (111, 311), and wherein the exit end (114, 314) of the needle hub (110, 310) is provided with a second adhesive (112, 312).

2. A medical glass syringe (100, 300) according to claim 1, wherein the second adhesive (112, 312) has generally no oxygen inhibiting properties and/or wherein the first adhesive (111, 311) has oxygen inhibiting properties.

3. A medical glass syringe (100, 300) according to any of the preceding claims, wherein when uncured the second adhesive (112, 312) has a lower viscosity than the first adhesive (111, 311).

4. A medical glass syringe (100, 300) according to any of the preceding claims, wherein the first and the second adhesive (112, 312) are curable with radiation of the same wavelength, wherein the wavelength of the radiation is in the range 320 nm to 410 nm, preferably in the range 340 nm to 390 nm, most preferably around 365 nm.

5. A medical glass syringe (100, 300) according to any of the preceding claims, wherein the first and the second adhesive (112, 312) have the same optical properties when cured.

6. A medical glass syringe (100, 300) according to any of the preceding claims, wherein when uncured, the weight ratio between the first and the second adhesive (112, 312) is in the range of 0.2 to 1.8, preferably in the range of 0.6 to 1.4, most preferably 1.

7. A medical glass syringe (100, 300) according to any of the preceding claims, wherein the second adhesive (112, 312) forms a dome-shaped structure on the exit end (114, 314) and wherein the height of the dome-shaped structure is preferably in the range of 1 mm to 5 mm, more preferably in the range of 2 mm to 4 mm, and most preferably around 3 mm and wherein the diameter of the dome-shaped structure is preferably in the range of 2 mm to 8 mm, more preferably in the range of 4 mm to 6 mm, and most preferably about 5 mm.

8. A medical glass syringe (100, 300) according to the preceding claim, wherein the shaft of the needle (102, 302) passes through the dome-shaped structure.

9. A medical glass syringe (100, 300) according to any of the preceding claims, wherein the first adhesive (111, 311) is LOCTITE^{®} AA 3345 and the second adhesive (112, 312) is preferably LOCTITE^{®} AA 3942.

10. A medical glass syringe (100, 300) according to any of the preceding claims, wherein the first adhesive (111, 311) is in communication with the interior space (106, 306) of the glass barrel (104, 304) and wherein the second adhesive (112, 312) is not in communication with the interior space (106, 306) of the glass barrel (104, 304).

11. A medical glass syringe (100, 300) according to any of the preceding claims, wherein the first adhesive (111, 311) is arranged close to the interior space (106, 306) of the glass barrel (104, 304), such that a medical drug inside the medical syringe is in contact with the first adhesive (111, 311) and not with the second adhesive (112, 312).

12. A medical glass syringe (100, 300) according to any of the preceding claims, wherein the barrel and the needle hub (110, 310) are integrally formed with each other.

13. A medical glass syringe (100, 300) according to any of the preceding claims, wherein the first adhesive (111, 311) is arranged in the needle hub (110, 310) as to extend to the exit end (114, 314) of the needle hub (110, 310), and as to be flush with the surface of the exit end (114, 314); and
wherein preferably the second adhesive (112, 312) completely covers the first adhesive (111, 311) at the surface of the exit end (114, 314).

14. A method of treating a medical glass syringe (100, 300) in accordance with any one of the preceding claims, comprising the curing of the first and the second adhesive (112, 312) by means of radiation.

15. A method according to claim 14, wherein the means of radiation does not comprise a mercury vapour lamp and wherein the means of radiation preferably comprises a LED, the LED preferably configured to emitting UVA (2000) radiation.
